# EUROPÄISCHE PATENTSCHRIFT

(11) **EP 1 134 001 B1**
(45) Veröffentlichungstag und Bekanntmachung des Hinweises auf die Patenterteilung: **07.06.2006**
(21) Anmeldenummer: 01104211.6
(22) Anmeldetag: 22.02.2001
(51) Int. Cl.: A61M 5/31

(54) **Verwendung einer Spritze für medizinische Zwecke in Verbindung mit einer Transportvorrichtung**
Use of a medical syringe in connection with a transport device
Utilisation d'une seringue médicale reliée à un dispositif de transport

(30) Priorität: 15.03.2000 DE 10012574
(43) Veröffentlichungstag der Anmeldung: 19.09.2001
(73) Patentinhaber: Schott AG, 55122 Mainz (DE)
(72) Erfinder: Heinz, Jochen, Dr., 55578 Vendersheim (DE); Spallek, Michael, Dr., 55218 Ingelheim (DE); Fabian, Arthur, 55131 Mainz (DE)
(74) Vertreter: Fuchs Mehler Weiss & Fritzsche

(56) Entgegenhaltungen:
- DE-B- 1 059 151
- US-A- 2 562 129
- US-A- 5 980 495

## Beschreibung

Die Erfindung geht aus von einer Spritze für medizinische Zwecke, mit einem Spritzenkörper, an dem austrittseitig ein verschließbarer Spritzenkopf angeformt ist, und der am anderen Ende eine Fingerauflage aufweist, die in zwei spiegelsymmetrisch gegenüberliegenden Bereichen eine vergrößerte Auflagefläche besitzt, wobei die beiden Bereiche eine parabelförmige Konfiguration haben, deren Scheitel jeweils vom Spritzenkörper weg gerichtet ist und die im Bereich des Spritzenkörpers bogenförmig ineinander übergehen.
Eine Spritze mit einer derartigen Fingerauflage ist durch mehrere Schriften bekannt geworden (US-A-2 562 129; DE 38 11 973 C1; DE 1 059 151 B; US-A-5 024 661 und US-A-2 566 428).

Die Erfindung betrifft eine spezifische Verwendung dieser Spritze in Verbindung mit einer Transportvorrichtung.

Spritzen für medizinische Zwecke im Sinne der Erfindung sind Spritzen, mit denen eine pharmazeutische Substanz oder eine diagnostisch wirksame Substanz durch Injektion parenteral verabreicht werden kann. Dabei gewinnen die vorfüllbaren Einmalspritzen, sogenannte Fertigspritzen, immer mehr an Bedeutung. Eine derartige Fertigspritze vermeidet das sonst übliche Überführen der medizinischen Substanz aus Ampullen und Injektionsflaschen in die Spritze. Dieses Überführen ist nicht nur zeitaufwendig, sondern es ermöglicht auch eine große Anzahl von Verunreinigungsquellen.

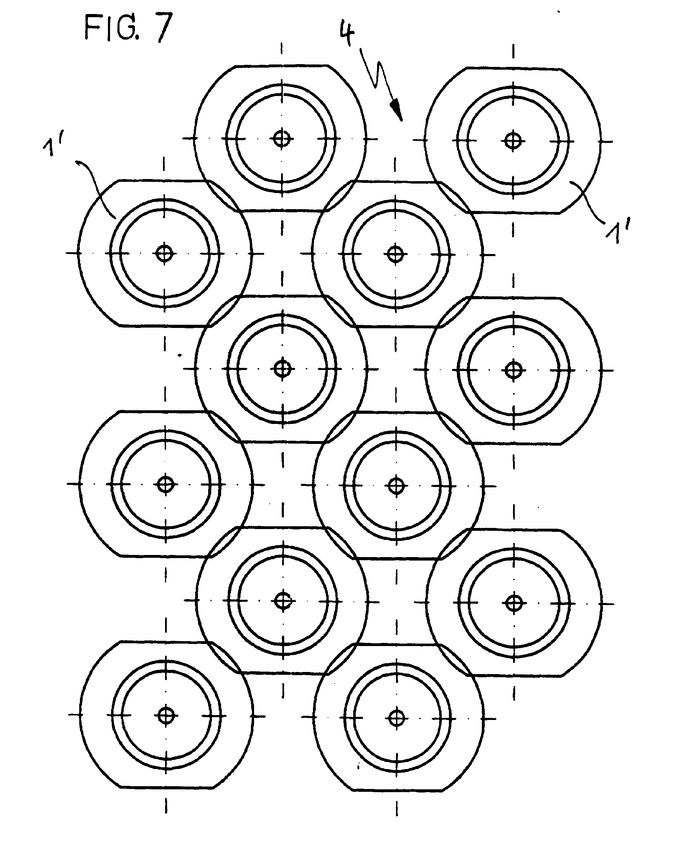

Vorfüllbare Einmalspritzen aus Röhrenglas sind Gegenstand der Norm DIN ISO 11040 Teil 4. Die Maße der Spritzen sind in dieser Norm festgelegt, ebenso ihre Konfiguration, die in der Fig. 5 im Längsschnitt dargestellt ist. Die Kopfausführung des Spritzenzylinders muß zwischen Hersteller und Abnehmer vereinbart werden. Der Kopf kann dabei einen Luer-Konus aufweisen, der mittels eines sogenannten Tip-Cap verschließbar ist und auf den nach Abnehmen des Tip-Cap ein Nadelaufsatz aufschiebbar ist. Der Kopf kann auch eine integrierte Nadel aufweisen, die mit einer konventionellen Nadelkappe abdeckbar ist.

An dem Spritzenzylinder ist eine Fingerauflage angeformt, deren durch vorgenannte Norm festgelegte Konfiguration aus der Fig. 6 ersichtlich ist. Sie hat eine kreisförmige Basis-Konfiguration, wobei in zwei gegenüberliegenden Bereichen jeweils ein Segment "abgeschnitten" ist, so daß in den beiden anderen um 90° versetzten gegenüberliegenden Kreisbogen-Bereichen eine relativ schmale, ergonomisch nicht optimale Auflage gegeben ist. Diese Auflage ist nämlich so schmal, daß es häufig beim Applizieren notwendig ist, die Fingerauflagefläche durch entsprechende Kunststoffteile, die am Spritzenzylinder angeclipt werden, zu vergrößern, wie es beispielsweise die EP 0 738 517 A 1 in den Figuren 18 bis 20 zeigt.

Ähnlich ist die Ausbildung der Fingerauflagen bei Einmalspritzen aus Kunststoff gemäß der Norm DIN 13 098 Teil 1 sowie für vorfüllbare Spritzen aus Kunststoff, wie sie beispielsweise aus der DE 44 38 360 C 1 bekannt geworden sind.

Neben dem Nachteil der ungünstigen ergonomischen Gestaltung der Fingerauflagen kommt noch ein weiterer durch die Konfiguration der Fingerauflage bedingter Nachteil hinzu.

Die Spritzen für medizinische Zwecke werden typischerweise zunächst in einem Glas bzw. Kunststoff verarbeitenden Betrieb hergestellt und danach zu einem pharmazeutischen Betrieb transportiert, wo sie befüllt werden. Sowohl bei der Herstellung der Spritzen als auch bei ihrer Befüllung sind eine Reihe von Bearbeitungsschritten notwendig, wie Waschen, Sterilisieren, Verpacken, Transportieren, Silikonisieren, Konfektionieren etc. So ist beispielsweise die Verarbeitung von Fertigspritzen - dort Spritzenampullen genannt - aus dem Aufsatz von E. Venten und J. Hoppert "Pharmazeutische Industrie" 40 Nr. 6, 1978, Seite 665 - Seite 671 sowie aus "Die neue Verpackung", Band 31, 7/1978, Seiten 1062 - 1074, bekannt.

Besondere Probleme bereitet hierbei die Handhabung, der Transport und die Lagerung von solchen befüllten wie unbefüllten Spritzen. Ein wesentlicher Grund liegt darin, daß diese Spritzen einen Massenartikel darstellen und daher insoweit nicht einzeln gehandhabt werden können, sondern aus wirtschaftlichen Gründen sozusagen nur in geordneten Chargen bzw. magaziniert in sogenannten Trays maschinell gehandhabt werden können.

Entsprechende Vorrichtungen sind in zahlreichen Varianten bekannt geworden (WO 94/14484 und EP 0 790 063 A 1 mit dem HYPAK® SCF®-System). Diese Vorrichtungen weisen typischerweise starre Kunststoff-Lochplatten, sogenannte "Nester", auf, in denen die Spritzen, an ihren Fingerauflagen hängend, lose gehalten werden.

Um eine möglichst effiziente Verarbeitung der Spritzen zu gewährleisten, muß jedoch eine möglichst hohe Packungsdichte der Spritzen erzielt werden, was mit den bekannten Systemen aufgrund der Konfiguration der Fingerauflagen nur unzureichend gelingt, wie aus der Fig. 7 unmittelbar ersichtlich ist.

Der Erfindung liegt die Aufgabe zugrunde, bei Handhabung, Transport und Lagerung von medizinischen Spritzen eine sehr hohe Packungsdichte beim Magazinieren in Lochplatten zu erzielen. Die Lösung dieser Aufgabe gelingt durch die Verwendung einer Spritze für medizinische Zwecke, mit einem Spritzenkörper, an dem austrittseitig ein verschließbarer Spritzenkopf angeformt ist, und der am anderen Ende eine Fingerauflage aufweist, die in zwei spiegelsymmetrisch gegenüberliegenden Bereichen eine vergrößerte Auflagefläche besitzt, wobei die beiden Bereiche eine parabelförmige Konfiguration haben, deren Scheitel jeweils vom Spritzenkörper weg gerichtet ist und die im Bereich des Spritzenkörpers bogenförmig ineinander übergehen, in Verbindung mit einer Transportvorrichtung, bestehend aus einer biegesteifen Trägerplatte aus geschäumtem Kunststoff, die mehrere versetzt zueinander angeordnete Lochreihen aufweist, indem die Spritzen mit dem Spritzenkopf nach unten an der Fingerauflage hängend in den Löchern der Lochreihen aufgenommen werden, für die Magazinierung der Spritze.

Durch die Verwendung der eingangs bezeichneten Spritzen mit der speziell konfigurierten Fingerauflage - auch Griffleiste genannt - für die Magazinierung in einer speziellen Trägerplatte ist eine dichte Packung der leeren oder befüllten Spritzen möglich. Verglichen mit entsprechenden Spritzen nach DIN-ISO 11040-4 ergibt sich eine bedeutend erhöhte Packungsdichte der erfindungsgemäß verwendeten Spritzen.

Ferner ist die Fingerauflage der erfindungsgemäß verwendeten Spritze ergonomisch wesentlich günstiger, da sie eine relativ große Auflagefläche besitzt, die eine hohe Griffsicherheit gewährleistet.
Dabei ist die Fingerauflage zweckmäßig einstückig an dem Spritzenkörper angeformt. Dadurch ist eine einfache Herstellung der Spritze möglich.
Prinzipiell kann jedoch auch die Fingerauflage ein separates Teil sein, das mit dem Spritzenkörper mit bekannten Methoden, z.B. Kleben, verbunden wird.

Die erfindungsgemäß verwendete Spritze kann aus Glas oder alternativ auch aus Kunststoff bestehen. Als Kunststoff eignet sich bekanntermaßen mit besonderem Vorteil ein Cycloolefincopolymer (COC) oder Cycloolefinpolymer (COP).

Die erfindungsgemäß verwendete Spritze kann zweckmäßig als Einmalspritze ausgebildet sein. Mit großem Vorteil kann sie auch als vorfüllbare Einmalspritze (Fertigspritze) ausgebildet sein.

Typischerweise ist der Spritzenkörper zylindrisch ausgebildet. Allerdings sind auch Spritzen mit einer von der Kreisform abweichenden Querschnittskonfiguration denkbar.

Die erfindungsgemäß verwendete Spritze ist zweckmäßig so ausgebildet, daß die maximalen äußeren radialen Abmessungen des Spritzenkopfes, gegebenenfalls einschließlich eines zugehörigen Verschlusses, nicht größer sind als die maximale radiale Abmessung des Spritzenkörpers. Eine derartige Spritze ist an sich durch die US-A-5 980 495 bekannt geworden. Aufgrund dieser Ausbildung ist es möglich, die Spritze, mit dem Kopf nach unten mittels der Fingerauflage in der Trägerplatte hängend, magaziniert zu handhaben, auch dann, wenn der Spritzenkopf bereits verschlossen ist und über das andere offene Ende die Spritze befüllt und dann mittels eines Kolbenstopfens verschlossen wird.

Weitere Ausgestaltungen der Erfindung ergeben sich aus der Beschreibung von in den Zeichnungen dargestellten Ausführungsbeispielen.

Es zeigen:
- Fig. 1: in einer Draufsichtdarstellung die speziell konfigurierte Fingerauflage einer erfindungsgemäß verwendeten Einmalspritze,
- Fig. 2: in einer schematischen perspektivischen Darstellung den Grundaufbau einer Trägerplatte für den Sammel-Transport der erfindungsgemäß verwendeten Spritzen,
- Fig. 3: in vier Figurenteilen A - D Querschnittsansichten von vier verschiedenen Ausbildungen des Klemmsitzes zur Halterung der Spritzen in den Bohrungen der Trägerplatte,
- Fig. 4: eine schematische Draufsicht auf die mit Spritzen nach der erfindungsgemäßen Verwendung bestückte Trägerplatte,
- Fig. 5: einen Längsschnitt durch eine genormte vorfüllbare Einmalspritze aus Glas,
- Fig. 6: eine Draufsicht auf die genormte Fingerauflage der Normspritze nach Fig. 5, und
- Fig. 7: eine schematische Draufsicht auf die mit Spritzen mit der genormten Fingerauflage bestückte Trägerplatte.

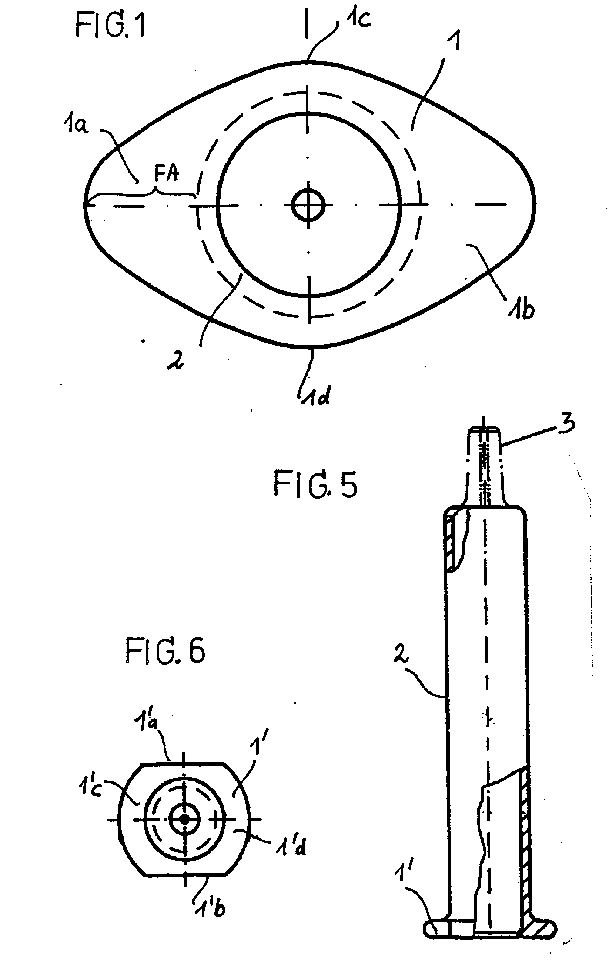

Die Fig. 1 zeigt in einer sehr vergrößerten Draufsicht-Darstellung die Konfiguration der Fingerauflage 1 für eine insbesondere vorfüllbare erfindungsgemäß verwendete Einmalspritze, die sich wesentlich von der Konfiguration der Fingerauflage 1' (Fig. 6) der bekannten normierten Fertigspritze, die in Fig. 5 in einem aufgebrochenen Längsschnitt dargestellt ist, unterscheidet. Die bekannte, normierte Fingerauflage nach Fig. 6, besitzt eine kreisförmige Basis-Konfiguration, wobei in zwei gegenüberliegenden Bereichen 1' a und 1' b jeweils ein Segment abgeschnitten ist, so daß in den beiden anderen, um 90° versetzten, gegenüberliegenden Kreisbogenbereichen 1' c und 1' d nur eine relativ schmale, ergonomisch nicht optimale Auflage gegeben ist, die auch keine optimale Packungsdichte in den sogenannten Nestern erlaubt.

Die bekannte normierte Spritze nach Fig. 5 besitzt im übrigen einen Spritzenzylinder 2, an dem ein Spritzenkopf 3 angeformt ist, wie auch die Fingerauflage 1' typischerweise an dem Spritzenzylinder 2 angeformt, d.h. einstückig mit ihm verbunden ist. Der Spritzenkopf 3 kann, wie dargestellt, durch einen Luer-Konus gebildet werden, der durch ein sogenanntes Tip Cap keim- und flüssigkeitsdicht verschließbar ist. Er kann auch eine integrierte Nadel, die mit einer Schutzkappe abgedeckt ist, aufweisen.

Die besondere, an sich ebenfalls bekannte Konfiguration der Fingerauflage der erfindungsgemäß verwendeten Spritze nach Fig. 1 besteht nun darin, daß sie auf zwei gegenüberliegenden Seiten mittensymmetrisch zwei Bereiche 1 a und 1 b mit parabelförmiger Kontur besitzt, wobei die beiden Bereiche mittig (Positionen 1 c , d) stetig bogenförmig ineinander übergehen. Dadurch steht die relativ große Strecke FA als Auflage für die Finger beim Applizieren zur Verfügung. Außerdem kann eine sehr hohe Packungsdichte beim Applizieren erzielt werden, wie noch zu erläutern sein wird.

Im übrigen entspricht, sofern es sich um eine Glas-Fertigspritze handelt, die Konfiguration der erfindungsgemäßen Spritze derjenigen nach Fig. 5, mit ebenfalls vorzugsweise einstückig angeformter Fingerauflage nach Fig. 1.

Die erfindungsgemäß verwendete Spritze besteht daher ebenfalls aus einem zylindrischen Spritzenkörper 2 und weist kopfseitig einen Verschluß auf, dessen maximaler Durchmesser nicht größer ist als der maximale Durchmesser des Spritzenkörpers, damit die Spritze in Lochhalterungen, mit dem Kopf nach unten in der Fingerauflage hängend, aufnehmbar ist. Der Spritzenkörper muß nicht zwingend zylindrisch sein, er kann auch, wie in der WO 99/22788 gezeigt, im Querschnitt mehreckig sein.

Sofern die erfindungsgemäß verwendete Spritze aus Kunststoff hergestellt werden soll, gilt entsprechendes.

Wie bereits eingangs erwähnt, werden die Spritzen der vorgenannten Art typischerweise in Lochplatten, mit dem Spritzenkopf nach unten in ihren Fingerauflagen hängend, magaziniert gehandhabt.

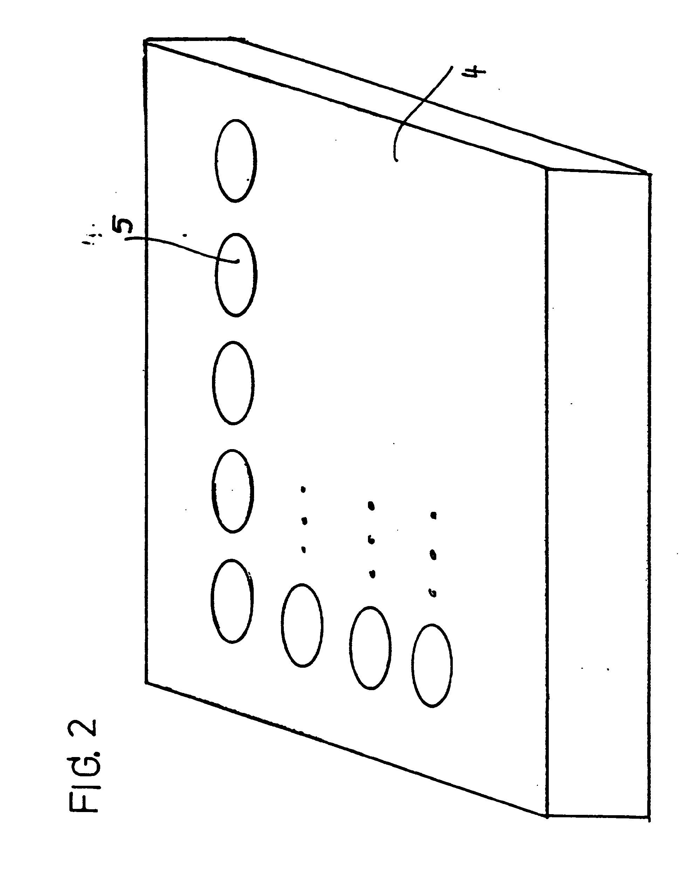

In Fig. 2 zeigt eine derartige Transportvorrichtung, die eine einstückige biegesteife, rechteckförmige Trägerplatte 4 aus geschäumten Kunststoff, vorzugsweise geschäumten Polypropylen (PP) oder Polyester (PET) oder ähnlichen Materialien aufweist, wie sie beispielsweise in dem Messebericht zur Tokyo Pack 1992 in "Neue Verpackung", 12/92 Seiten 32 - 38 beschrieben werden.

In der Trägerplatte 4 sind in mehreren Reihen, von denen nur eine Reihe komplett dargestellt ist, kreisrunde Bohrungen 5 oder auch Bohrungen mit mehreckigem Querschnitt zur Aufnahme der Spritzen 2 ausgebildet. Diese Bohrungen 5 werden zweckmäßig bereits beim Herstellen der Trägerplatte ausgeformt.

Vorzugsweise ist die Trägerplatte 4 so ausgebildet, daß sie eine porenfreie Oberfläche besitzt. Dies kann typischerweise durch eine nachträgliche Oberflächenbehandlung der geschäumten Trägerplatte geschehen.

Der Durchmesser der Bohrungen 5 ist so auf den Durchmesser des Spritzenzylinders 2 abgestimmt, daß die Behälter klemmend und zentriert in der Bohrung aufgenommen sind. Dadurch verhindert die bei der erfindungsgemäßen Verwendung der Spritzen eingesetzte Transportplatte Relativbewegungen zwischen dem Spritzenkörper und der Trägerplatte 4 sicher und ist somit auch für verkratzungsempfindliche Spritzen, z.B. Spritzen aus spröden Kunststoffen, geeignet.

Die sichere Führung der Spritzen hat darüber hinaus den Vorteil, daß es nicht durch Relativbewegungen zwischen Spritze und Trägerplatte (Nest) zu statischen Aufladungen und zur Partikelbildung kommen kann.

Weiterhin verhindert die sichere Führung, daß die beim Transport unvermeidlichen Rüttelbewegungen dazu führen, daß nach oben hüpfende Spritzen die Siegelfolie, die zum Verschluß des gesamten Behältnisses dient, durchscheuern oder beschädigen, und damit die Gefahr von Unsterilitäten entsteht. Um dieses zu verhindern, werden beim Stand der Technik schützende Zwischenfolien oder Zwischenschäume eingesetzt, welche durch die bei der erfindungsgemäßen Verwendung vorgesehene Trägerplatte überflüssig werden.

Die Träger- bzw. Transportplatte kann, sofern sie diese Fixierung gewährleistet, auch zur direkten Befüllung benutzt werden, bei der dann im Gegensatz zur heutigen Praxis die Spritzen nicht mehr einzeln oder gruppenweise aus der Platte gehoben und aufwendig zur Abfüllung zentriert und ausgerichtet werden müssen. Es können daher mit dem erfindungsgemäßen magazinierten Nest gegenüber dem heutigen Stand der Technik erheblich höhere Abfülleistungen erzielt werden.

Die beschriebene Transportplatte kann - wenn sie eine geschlossene porige Oberfläche aufweist - mit Vorteil zusammen mit leeren oder befüllten Spritzen autoklaviert werden, so daß für diesen Sterilisierungsschritt kein "Umladen" der Behälter notwendig ist.

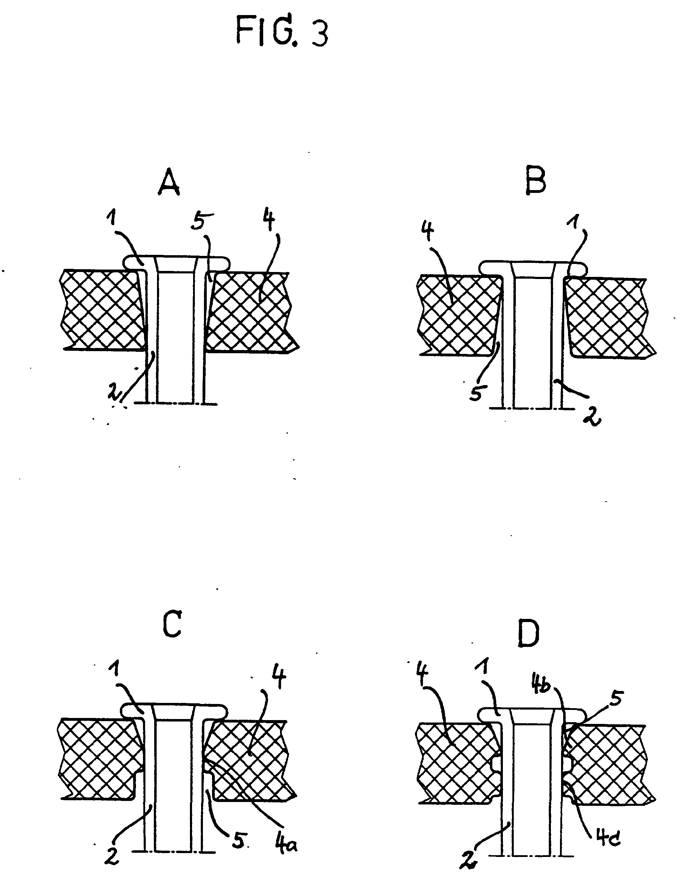

In der Figur 3 ist eine Querschnittansicht der Trägerplatte 4 in der Umgebung einer Bohrung 5 dargestellt.

Wie in Fig. 3 in den Figurtenteilen A und B für Spritzenkörper 2, deren Fingerauflage 1 in der Bohrung 5 über die Trägerplatte 4 übersteht, dargestellt ist, sind die Bohrungen 5 vorzugsweise konisch ausgeführt, so daß die eingesteckten Spritzenkörper sicher fixiert und zentriert sind. So werden Toleranzen in der Spritzenkörpergeometrie ausgeglichen.

Dabei kann sich die konische Fläche nach unten (Fig. 3 A) oder nach oben verjüngen (Fig. 3 B).

Der Spritzenkörper 2 kann in der Öffnung 5 der Trägerplatte 4 auch, wie in den Figuren 3 C dargestellt, in der Weise klemmend gehalten werden, daß in der Öffnung 5 an der Trägerplatte 4 ein umlaufender ringförmiger Fortsatz 4 a angeformt ist, mit einem Durchmesser an seiner engsten Stelle, der etwas kleiner als der Durchmesser des Spritzenzylinders 2 ist.

Bei der Ausführungsform nach der Figur 3 D sind dabei zwei axial beabstandete umlaufende ringförmige Fortsätze 4 b und 4 c angeformt, was den sicheren Halt noch verbessert.

In einer besonderen Ausführungsform der Transportplatte sind die Bohrungen 5 so gestaltet, daß die Führung und Klemmung der Spritzenkörper 2 an drei Kanten (punktuelle Fortsätze) erfolgt, was den selbstzentrierenden Effekt verbessert, und eine thermische Isolation eines bestimmten Spritzenkörperabschnittes verhindert. Diese Kanten befinden sich vorzugsweise auf unterschiedlichen axialen Höhen.

Alternativ zu der Ausbildung nach Fig. 3 können die Bohrungen 5 auch so ausgebildet sein, daß in der Transportplatte die Fingerauflagen 1 versenkt liegen.

In einer besonderen Ausführungsform besteht die Transportplatte 4 aus geschäumtem Kunststoff, der mit in Inertgasen (z.B. Stickstoff, Kohlendioxid, Argon) aufgeschäumt wurde. Dies verhindert sicher die Bildung von Ozongas bei der Sterilisation durch energiereiche Strahlung (ä-Strahlung oder Elektronenstrahlung).

Gemäß einer Ausgestaltung besteht die Transportplatte aus geschäumtem Kunststoff, der versteifende Elemente enthält.

In einer alternativen Ausführungsform weist die Transportplatte eine Rippengeometrie zur Versteifung auf.

Die Trägerplatte 4 kann bei den unterschiedlichsten Handhabungsvorgängen beim Herstellen und Befüllen der Spritzen eingesetzt werden. Sie ist auch als Einsatz in einem wannenförmigen Behälter als Transportverpackung für leere oder befüllte Spritzen einsetzbar.

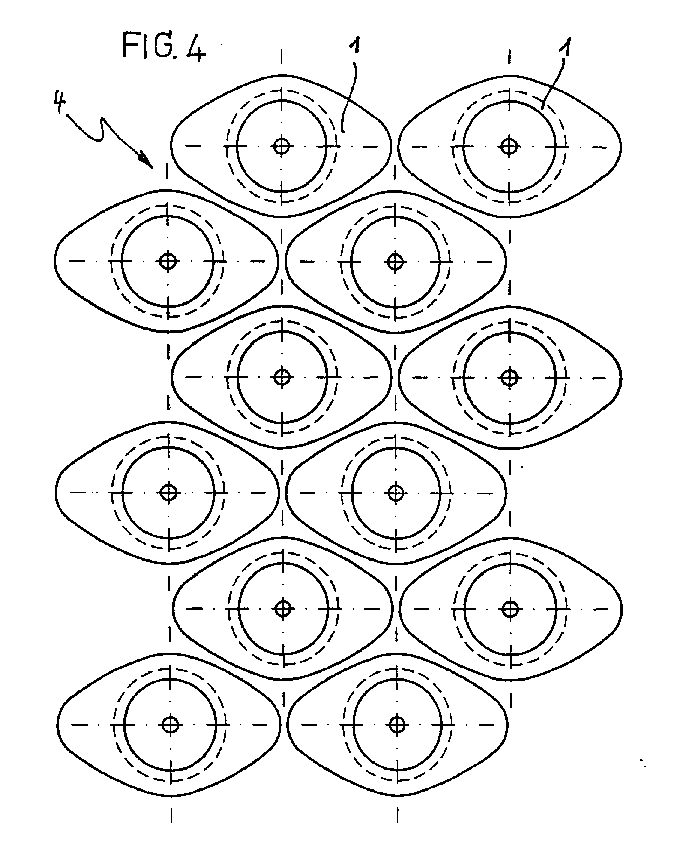

Die Figur 4 zeigt eine Ausbildung der Trägerplatte 4 mit mehreren, versetzt zueinander angeordneten Lochreihen, so daß mit den erfindungsgemäß verwendeten Spritzen aufgrund der speziell gestalteten, mittensymmetrisch parabelförmigen Fingerauflage 1 erkennbar eine sehr hohe Packungsdichte erreicht werden kann, was die Wirtschaftlichkeit bei der Herstellung und Befüllen der Spritzen erhöht und somit die Kosten senkt. Diese hohe Packungsdichte im Vergleich zu den Normspritzen ergibt sich visuell durch den Vergleich der Fig. 4 mit der Fig. 7, die erhebliche Lücken zwischen den genormten Fingerauflagen 1' erkennen läßt.

## Patentansprüche

1. Verwendung einer Spritze für medizinische Zwecke, mit einem Spritzenkörper (2), an dem austrittsseitig ein verschließbarer Spritzenkopf (3) angeformt ist, und der am anderen Ende eine Fingerauflage (1) aufweist, die in zwei spiegelsymmetrisch gegenüberliegenden Bereichen eine vergrößerte Auflagefläche besitzt, wobei die beiden Bereiche (1 a, 1 b) eine parabelförmige Konfiguration haben, deren Scheitel jeweils vom Spritzenkörper (2) weg gerichtet ist und die im Bereich (1 c, 1 d) des Spritzenkörpers (2) bogenförmig ineinander übergehen, in verbindung mit einer Transportvorrichtung, bestehend aus einer biegesteifen Trägerplatte (4) aus geschäumtem Kunststoff, die mehrere versetzt zueinander angeordnete Lochreihen aufweist, indem die Spritzen mit dem Spritzenkopf (3) nach unten an der Fingerauflage (1) hängend in den Löchern (5) der Lochreihen aufgenommen werden, für die Magazinierung der Spritze.

2. Verwendung nach Anspruch 1 bei der die Fingerauflage (1) der Spritze einstückig an dem Spritzenkörper (2) angeformt ist.

3. Verwendung nach Anspruch 1 oder 2, bei der die Spritze aus Glas besteht.

4. Verwendung nach Anspruch 1 oder 2, bei der die Spritze aus einem Kunststoff besteht.

5. Verwendung nach Anspruch 4, bei welcher der Kunststoff der Spritze ein Cycloolefincopolymer (COC) oder ein Cycloolefinpolymer (COP) ist.

6. Verwendung nach einem der Ansprüche 1 bis 5, bei welcher die Spritze als Einmalspritze ausgebildet ist.

7. Verwendung nach einem der Ansprüche 1 bis 5, bei welcher die Spritze als vorfüllbare Einmalspritze ausgebildet ist.

8. Verwendung nach einem der Ansprüche 1 bis 7, bei welcher der Spritzenkörper (2) der Spritze zylindrisch ausgebildet ist.

9. Verwendung nach einem der Ansprüche 1 bis 8, bei welcher die maximalen äußeren radialen Abmessungen des Spritzenkopfes (3) der Spritze gegebenenfalls einschließlich eines zugehörigen Verschlusses, nicht größer sind als die maximale radiale Abmessung des Spritzenkörpers der Spritze.

## Claims

1. Use of a medical syringe, with a syringe body (2) on which a closable syringe head (3) is integrally formed at the discharge end and which, at the other end, has a finger support (1) comprising an enlarged support surface in two regions lying opposite one another in mirror symmetry, the two regions (la, 1b) having a parabolic configuration, whose vertex is in each case directed away from the syringe body (2), and merging into one another in an arcuate shape in the region (1c, 1d) of the syringe body (2), in connection with a transport device composed of a flexurally stiff carrier plate (4) which is made of foamed plastic and has several rows of holes arranged offset from one another, and the syringes, with the syringe head (3) pointing downwards, are suspended via the finger support (1) in the holes (5) of the rows of holes, for storing the syringes.

2. Use according to Claim 1, in which the finger support (1) of the syringe is formed in one piece on the syringe body (2).

3. Use according to Claim 1 or 2, in which the syringe is made of glass.

4. Use according to Claim 1 or 2, in which the syringe is made of a plastic.

5. Use according to Claim 4, in which the plastic of the syringe is a cycloolefin copolymer (COC) or a cycloolefin polymer (COP).

6. Use according to one of Claims 1 to 5, in which the syringe is designed as a disposable syringe.

7. Use according to one of Claims 1 to 5, in which the syringe is designed as a prefillable disposable syringe.

8. Use according to one of Claims 1 to 7, in which the syringe body (2) of the syringe has a cylindrical configuration.

9. Use according to one of Claims 1 to 8, in which the maximum external radial dimensions of the syringe head (3) of the syringe, if appropriate including an associated closure piece, are not greater than the maximum radial dimension of the syringe body of the syringe.

## Revendications

1. Utilisation d'une seringue pour des applications médicales, avec un corps de seringue (2), sur lequel une tête de seringue (3) pouvant être fermée est formée du côté sortie, et qui comporte à l'autre extrémité une plaque d'appui de doigt (1), qui présente une face d'appui agrandie dans deux régions symétriquement opposées, dans laquelle les deux régions (la, 1b) ont une forme parabolique dont le sommet est orienté à l'opposé du corps de seringue (2) et qui se raccordent l'une à l'autre par des arcs dans la région (1c, 1d) du corps de seringue (2), reliée à un dispositif de transport se composant d'une plaque de support rigide en flexion (4) en matière plastique cellulaire, qui présente plusieurs séries de trous disposés en décalage l'un par rapport à l'autre, dans laquelle les seringues sont suspendues avec la tête de seringue (3) vers le bas par la plaque d'appui de doigt (1) dans les trous (5) de la série de trous, pour le rangement de la seringue.

2. Utilisation selon la revendication 1, dans laquelle la plaque d'appui de doigt (1) de la seringue est formée d'une pièce avec le corps de seringue (2).

3. Utilisation selon la revendication 1 ou 2, dans laquelle la seringue est constituée de verre.

4. Utilisation selon la revendication 1 ou 2, dans laquelle la seringue est constituée d'une matière plastique.

5. Utilisation selon la revendication 4, dans laquelle la matière plastique de la seringue est un copolymère de cyclooléfine (COC) ou un polymère de cyclooléfine (COP).

6. Utilisation selon l'une quelconque des revendications 1 à 5, dans laquelle la seringue est une seringue à usage unique.

7. Utilisation selon l'une quelconque des revendications 1 à 5, dans laquelle la seringue est une seringue à usage unique pouvant être préremplie.

8. Utilisation selon l'une quelconque des revendications 1 à 7, dans laquelle le corps de seringue (2) de la seringue est cylindrique.

9. Utilisation selon l'une quelconque des revendications 1 à 8, dans laquelle les dimensions radiales extérieures maximales de la tête de seringue (3) de la seringue, y compris éventuellement une fermeture correspondante, ne sont pas plus grandes que la dimension radiale maximale du corps de seringue de la seringue.
